Europäisches Patentamt

European Patent Office

Office européen des brevets

(11) Veröffentlichungsnummer: **0 205 851**
**B1**

(12) **EUROPÄISCHE PATENTSCHRIFT**

(45) Veröffentlichungstag der Patentschrift:
28.02.90

(51) Int. Cl.⁴: **A61M 25/00**, **A61B 17/38**,
**A61M 29/02**

(21) Anmeldenummer: 86106233.9

(22) Anmeldetag: 07.05.86

(54) Katheter.

(30) Priorität: 10.05.85 DE 3516830

(43) Veröffentlichungstag der Anmeldung:
30.12.86 Patentblatt 86/52

(45) Bekanntmachung des Hinweises auf die Patenterteilung:
28.02.90 Patentblatt 90/9

(84) Benannte Vertragsstaaten:
AT BE CH DE FR GB IT LI NL SE

(56) Entgegenhaltungen:
WO-A-84/03829
DE-A- 2 315 075
DE-A- 3 014 236
DE-A- 3 315 303
DE-A- 3 428 644
GB-A- 2 115 289
US-A- 2 801 553
US-A- 3 324 847
US-A- 3 952 742
US-A- 4 038 519

(73) Patentinhaber: Hubmann, Max, Dr., Rathsberger Strasse 24, D-8520 Erlangen(DE)
Patentinhaber: Thull, Roger, Prof. Dr.-Ing., Habichtweg 9, D-8551 Hemhofen(DE)

(72) Erfinder: Hubmann, Max, Dr., Rathsberger Strasse 24, D-8520 Erlangen(DE)
Erfinder: Thull, Roger, Prof. Dr.-Ing., Habichtweg 9, D-8551 Hemhofen(DE)

(74) Vertreter: Hufnagel, Walter, Dipl.-Ing., Dipl.-Wirtsch.-Ing. et al, Dorner & Hufnagel Patentanwälte Bad Brückenauer Str. 19, D-8500 Nürnberg 90(DE)

## Beschreibung

Die vorliegende Erfindung bezieht sich auf einen Katheter gemäß dem Oberbegriff des Anspruches 1.

Ein derartiger Katheter ist aus der DE-A 23 11 807 bekannt. Dieser Katheter besitzt zwei getrennte Lumen, von denen das eine am vorderen Ende des Katheters in einer Spülöffnung endet. Die das andere Lumen begrenzende Wandung besitzt Öffnungen, die in einen den Katheter im vorderen Endbereich umgebenden Ring aus Schaumstoff münden. Der Schaumstoffring kann durch eine durch das Lumen zugeführte Flüssigkeit zum Anschwellen gebracht werden, so daß der Katheter in einem Gewebekanal festgehalten werden kann. Es sind auch Katheter bekannt, bei denen ein aufblasbarer Gummiballon verwendung findet.

Aus der DE-A- 33 15 303 ist ein Katheter bekannt, der im Bereich des vorderen Endes einen Wärmeapplikator in Form einer dünnwandigen Hülse aus kompaktem Material, beispielsweise Edelstahl, aufweist. Diese Hülse ist durch den als Koaxialkabel ausgebildeten Katheter mit einer äußeren Spannungsquelle verbunden. Der Katheter ist dabei nicht mit einer Öffnung versehen und er besitzt keinerlei Kanäle, über die ein Behandlungsmittel zu- oder abgeführt werden kann. Auch ist die Hülse nicht flexibel, so daß der Anwendungsbereich dieses Katheters dadurch stark begrenzt ist. Die starre Hülse liegt nur in entsprechend engen Gewebe- oder Gefäßstellen mit einer zylindrischen Öffnung allseitig an diesen an. In zu behandelnden Körperteilen, deren Öffnungsquerschnitt größer ist als der Hülsendurchmesser, liegt die Hülse keinesfalls allseitig, allenfalls an undefinierten Stellen an.

Weiterhin ist aus der WO 84/03829 (DE 34 90 139 T 1) ein ähnlich wie ein Katheter einsetzbares chirurgisches Instrument bekannt, das aus einem dünnen biegsamen Silberrohr besteht, das mit Ausnahme des Endes mit einer elektrisch isolierenden Schicht überzogen ist. Über das Rohr kann Flüssigkeit abgesaugt werden und mit dem nicht isolierten Ende kann, wenn am Körper eine weitere Elektrode großflächig aufgebracht wird, mit Hilfe eines Stromes die Blutung einer Ader gestillt werden. Für die Behandlung von Gewebeteilen oder Gefäßen gelten dieselben Nachteile, wie im Zusammenhang mit der DE-A- 33 15 303 im einzelnen ausgeführt wurde.

Mit der vorliegenden Erfindung soll die Aufgabe gelöst werden, einen Katheter der eingangs beschriebenen Art so zu verbessern, daß in einem Körperteil, insbesondere in einem Gefäß, nur die zu behandelnde Stelle praktisch unabhängig von deren Querschnitt oder räumlichen Ausgestaltung einer intensiven Wärme- oder Hitzebehandlung durch elektrische Energie ausgesetzt werden kann.

Gelöst wird diese Aufgabe durch die im Kennzeichen des Anspruches 1 angegebenen Merkmale.

Durch die Anordnung des elektrisch leitenden Bereiches auf einer dehnbaren und aufweitbaren Unterlage, die vom Katheter selbst oder von einem Zwischenglied gebildet sein kann, kann durch ein durch den Kanal des Katheters geleitetes Druckmittel der beschichtete, elektrisch leitende Bereich erweitert werden, so daß dieser Bereich gegen die zu behandelnde Stelle gedrückt wird. Falls der elektrisch leitende Bereich nur an einer bestimmten Umfangsfläche des Katheters vorgesehen sein sollte, so kann die Erwärmung auf eine entsprechend kleine Stelle beschränkt werden. Die genaue Positionierung durch Drehen und Längsverschiebung des Katheters kann durch eine Röntgenbeobachtung vorgenommen werden, da der leitende Bereich oder ein Bereich aus einem röntgenkontrastreichen Material sich deutlich von den übrigen Umgebungsmaterialien abhebt.

Vorteilhafte Weiterbildungen des Katheters gemäß der vorliegenden Erfindung sind in den Unteransprüchen angegeben.

Der erfindungsgemäße Katheter kann gut sichtbar an die gewünschte Stelle innerhalb eines Blutgefäßes oder eines sonstige Körperflüssigkeiten enthaltenden Gefäßes herangeschoben werden. Durch den elektrisch leitfähigen Bereich kann beispielsweise hochfrequente Energie induktiv und/oder kapazitiv in das Körperinnere eingekoppelt werden. Es ist aber auch möglich, mittels einer im Katheter oder am Katheter angebrachten Zuleitung eine elektrische Spannung, insbesondere im Hochfrequenzbereich, direkt dem leitenden Behandlungsbereich zuzuführen. Je nach der eingekoppelten oder zugeführten Leistung kann zu therapeutischen Zwecken eine Erwärmung, ein Verkochen, Verdampfen, Koagulieren, Destrukturieren oder auch der Abbau von mechanischen Spannungen in Geweben, in Stenosen bildenden Materialien oder in anderen biologischen Strukturen an ganz bestimmten Stellen im Körper herbeigeführt werden. Der erfindungsgemäße Katheter kann hierbei durch vorhandene Körperöffnungen, intravenös, intraarteriell oder in anderer Weise eingeführt werden. Insbesondere kann mit dem erfindungsgemäßen Katheter die vielfach angewandte Ballondilatation in dem Sinne verbessert werden, daß Restenonisierungen innerhalb der dilatierten Bereiche weitestgehend vermieden werden können. Auch wenn die Dilatation ohne größere Intimaschädigung durchgeführt wird, so führt die im stenotischen Material vorhandene Elastizität regelmäßig zu einer gewissen Rückstellung, die die Restenosierung zumindest fördert. Diesem Vorgang wirkt die Behandlung mit dem erfindungsgemäßen Katheter unter Verwendung elektrischer Energie, vorzugsweise hochfrequenter Schwingungen, wirksam entgegen.

Weitere Einzelheiten und Vorteile der Erfindung werden nachfolgend anhand der in der Zeichnung veranschaulichten Ausführungsbeispiele näher beschrieben. Dabei zeigen:

Figur 1 einen katheter mit einer elektrisch leitenden Schicht auf einem aufblasbaren Ballon im Behandlungsbereich,

Figur 2 einen Katheter mit zumindest außen leitender Hülse,

Figuren 3 und 4 je einen Katheter gemäß Figuren 1 und 2 mit einer äußeren, elektrisch leitenden Zuleitung,

Figuren 5 und 6 je einen Katheter mit einer inneren, elektrisch leitenden Zuleitung,

Figuren 7 und 8 je einen Katheter mit zwei leitenden Bereichen und zwei elektrisch leitenden Zuleitungen und

Figur 9 einen Katheter gemäß Figur 1 mit einer Zwischenschicht zwischen dem Ballon und der elektrisch leitenden Schicht.

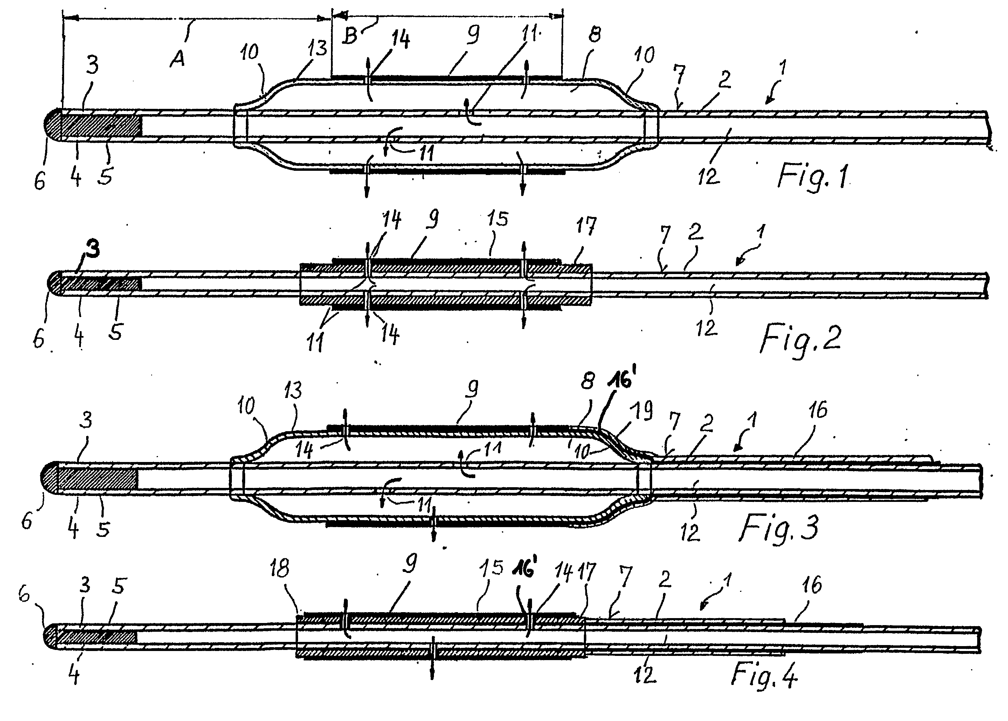

Der Katheter 1 gemäß Figur 1 besteht aus einem Rohr 2 aus insbesondere flexiblem Material. Dieses Rohr 2 ist am vorderen Ende 3 gas- und flüssigkeitsdicht verschlossen. Beispielsweise geschieht dies durch einen Einsatz 4 der am Ende 3 in das Rohr 2 eingeklebt, eingeschweißt, eingeschrumpft oder in sonstiger Weise befestigt ist. Der Einsatz 4 besteht hier beispielsweise aus einem Schaft 5 mit einem den Katheter 1 vorne abschließenden Rundkopf 6.

Das Ende des Katheters 1 ist mit einem Material abgedichtet, das für Röntgenstrahlen weniger gut durchlässig ist als beispielsweise Körpergewebe oder Knochen, so daß das Einführen des Katheters 1 in Blutgefäße oder andere Körperflüssigkeit führende Gefäße durch Röntgenstrahlen mit einem Beobachtungsschirm oder mit einem Monitor leicht verfolgt werden kann. Hierzu kann das Ende 3 des Katheters 1 innen und/oder außen metallisiert oder mit einem leitenden Kunststoff versehen sein oder aber der Einsatz 4 besteht aus Kontrastmaterial. Der Einsatz 4 oder die Wandung 7 des Katheters 1 können auch ein solches Material, wie beispielsweise Metall, Metallegierungen, Bariumsulfat, Calciumsulfat oder dgl. enthalten. Weiterhin ist in relativ kurzem Abstand A vom Ende 3 des Katheters 1 ein Behandlungsbereich B vorgesehen. In diesem Behandlungsbereich B ist gemäß Figur 1 auf ein den Katheter 1 dort umgebendes, als aufblasbarer Ballon 8 ausgebildetes Zwischenglied ein elektrisch leitender Bereich 9 in Form einer beispielsweise aufgedampften Metallschicht oder eines elektrisch leitfähigen Kunststoffes vorgesehen. Der Ballon 8 besteht aus einem flexiblen Schlauchabschnitt aus einem Material, das relativ hohe Temperaturen, beispielsweise bis zu etwa 300° C, kurzfristig aushält, wie beispielsweise Silikonkautschuk oder aus Polytetrafluorkohlenstoffen, vorzugsweise Polytetrafluoräthylen (PTFE). Der Ballon 8 ist an seinen beiden Enden 10 mit dem Rohr 2 dicht verbunden, insbesondere verklebt und/oder verschweißt. Innerhalb des Ballons 8 ist im Rohr 2 bzw. in der Katheterwand 7 wenigstens eine Öffnung 11 zum Aufblasen des Ballons 8 vorgesehen. Hierzu wird ein Druckmittel durch das Lumen 12 diesem Bereich des Katheters 1 zugeführt. Ein solcher Katheter 1 ist dazu geeignet, im Körperinneren therapeutisch so angewandt zu werden, daß um den Patienten eine elektrisch leitende Spule gelegt wird, die an eine Hochfrequenzspannung angeschlossen wird. Durch das sich ausbildende elektromagnetische Wechselfeld wird im leitenden Bereich 9 auf Grund von Wirbelströmen ein Strom induziert, durch den eine Erwärmung des den Bereich 9 umgebenden Materials, wie Gewebe, Stenosen oder dgl. eintritt. Je nach der eingekoppelten Energiemenge pro Zeiteinheit kann im Behandlungsbereich je nach den Erfordernissen eine Erwärmung, Verkochung, Verdampfung oder dergleichen der zu behandelnden Materialien, wie Gewebe, Stenosen oder dergleichen vorgenommen werden.

Um während der Behandlung ein Ankleben der leitenden Schicht 9 am Körpergewebe oder dgl. zu verhindern, sind in der Wand 13 des Ballons 8 oder/und im Bereich der leitenden Schicht 9 mehrere Öffnungen 14 vorgesehen, durch die das den Ballon 8 aufblasende Druckmittel nach außen zwischen das Gewebe und der leitenden Schicht 9 gedrückt werden kann. Gleichzeitig kann damit auch eine Kühlung des Ballons 8 erreicht werden.

Damit der Ballon 8 stets in aufgeblasenem Zustand gehalten werden kann, ist der Gesamtquerschnitt der Ballonöffnungen 14 kleiner als derjenige der Wandöffnungen 11, d.h. der Strömungswiderstand der Ballonöffnungen 11, d.h. der Strömungswiderstand der Ballonöffnungen 14 ist größer als derjenige der Wandöffnungen 11.

Als Druck- und/oder Spülmittel für den erfindungsgemäßen Behandlungskatheter 1 eignet sich jede physiologisch unbedenkliche Flüssigkeit. Vorzugsweise wird eine physiologische Kochsalzlösung verwendet.

Eine ähnliche Konstruktion eines Katheters 1 für Hochfrequenzerwärmung, wie anhand der Figur 1 beschrieben, zeigt die Figur 2. Dort ist anstelle des Ballons 8 als Zwischenglied eine Hülse 15 vorgesehen, auf deren Außenseite die leitende Bereiche 9 in Form einer leitenden Schicht vorgesehen ist. Auch diese Hülse 15 weist Öffnungen 14 auf, die mit denen der Katheteröffnungen 11 korrespondieren. Die Hülse 15 kann auch selbst elektrisch leitend ausgebildet sein. Günstigerweise besteht die Hülse 15 aus einem geeigneten Kunststoff, wie Silikonkautschuk, fluorierte Kohlenwasserstoffe, wie PTFE. Ggf. kann es vorteilhaft sein, die Hülse 15 aus schlecht wärmeleitendem Material herzustellen.

Die leitfähige Schicht 9 gemäß den Figuren 1 und 2 kann in den oben beschriebenen Ausführungsbeispielen eine beliebige Gestalt aufweisen. Sie kann vorzugsweise den Ballon 8 oder die Hülse 15 zylindrisch umgreifen oder aus nicht zusammenhängenden Segmenten, Streifen, Punkten oder dgl. bestehen. Einer kreisförmig in sich geschlossenen Form ist jedoch zur induktiven Einkopplung der Vorzug zu geben, vorzugsweise dann, wenn höhere Energien zur Anwendung gelangen sollen.

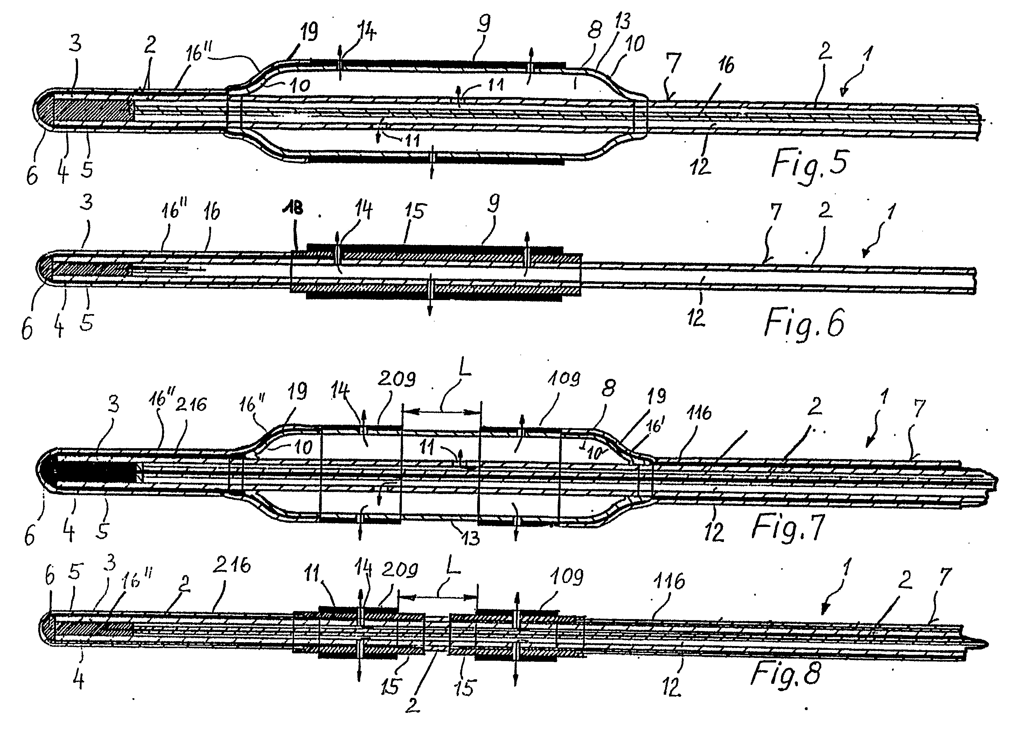

Gemäß den in den Figuren 3 bis 6 dargestellen Ausführungsbeispielen von denen die Figuren 3 und 5 je eine Ballonausführung und die Figuren 4 und 6 je eine Hülsenausführung zeigen, kann auch im oder am Katheter 1 eine elektrische Zuleitung 16 unmittelbar vorgesehen sein. Diese elektrische Zuleitung 16 ist gemäß den Figuren 3 und 4 am hinteren Ende 10 des Ballons 8 oder am hinteren Ende 17 der Hülse 15, also vor dem Behandlungsbereich B, über eine leitende Schicht 16' mit der leitenden Schicht 9 elektrisch leitend verbunden. In diesem Fall ist die Zuleitung 16 vorzugsweise auf dem Rohr 2 als Einzel- oder Koaxialleiter aufgebracht oder aber in die Wand 7 integriert. Sie kann jedoch auch gemäß den Fig. 5 und 6 im Katheter 1, insbesondere im Lumen 12, als isolierter Einzelleiter vorgesehen sein. Die Zuleitung 16 ist dann über das vordere Ende 3, insbesondere den elektrisch leitenden Einsatz 4, auf die Außenwand des Katheters 1 geführt und auf dieser als leitende Schicht 16" zum vorderen Ende 10 des Ballons 8 bzw. zum vorderen Ende 18 der Hülse 15 geführt. Nach außen ist sie mit einer Isolierschicht 19 versehen. Eine Isolierschicht 19 wird auch bei der Ausführung gemäß Figur 3 am hinteren Ende 10 des Ballons 8 vorgesehen.

Bei den Ausführungen mit der Hülse 15 kann die Zuleitung 16 grundsätzlich auch unterhalb der Hülse 15 verlaufen. Die Kontaktierung erfolgt in diesem Falle über die leitfähig gemachten Wandungen der Öffnungen 11 und 14, wie in den Figuren 4 und 6 durch eine dickere Strichstärke angedeutet ist, wenn nicht die Hülse 15 selbst elektrisch leitfähig ist.

Diese Ausführungsformen mit einer Zuleitung 16 eignen sich vorzugsweise zur kapazitiven Einkopplung der Hochfrequenzenergie. Hierzu wird der Patient beispielsweise auf eine leitende Platte gelegt, die die eine Elektrode bildet und an dem einen Pol der Spannungsquelle angeschlossen ist. Der andere Pol liegt über die Zuleitung 16 an der die zweite Elektrode bildenden leitenden Schicht 9. Hierdurch ist auch die zwischen den Elektroden liegende Körperpartie dem Hochfrequenzfeld ausgesetzt, was allein oder zusätzlich therapeutisch ausgenutzt werden kann.

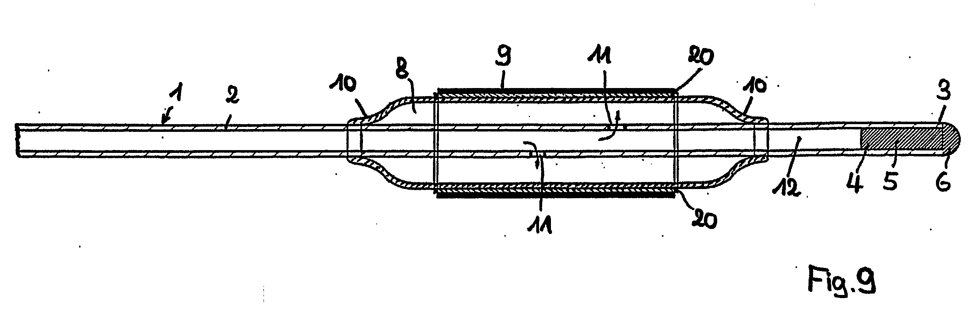

Eine andere Ausführungsform mit zwei Zuleitungen 116 und 216 ist für eine Ballonausführung in Figur 7 und für eine Hülsenausführung in Figur 8 dargestellt. Dort ist die leitende Schicht in zwei axial hintereinander angeordnete und im Abstand L voneinander entfernte lietende Schichten 109 und 209 aufgeteilt. Die erste leitende Schicht 109 ist mit der einen, insbesondere außen oder koaxial angeordneten Zuleitung 116 und die zweite leitende Schicht 209 ist mit der zweiten, insbesondere innen verlaufenden und über das vordere Ende 3 geführten Zuleitung 216 verbunden. Die Zuleitungen 116 und 216 werden am hinteren Ende des Katheters 1 mit der Spannungsquelle verbunden. Die therapeutisch wirksame Zone liegt hier vor allem in der dem Abstand L entsprechenden isolierenden Zone. Zur Erzielung eines optimalen Wirkungsgrades ist der Abstand L etwa gleich der Länge der elektrisch leitenden Schichten 109 und 209. Vorteilhaft ist bei der Ausführung gemäß Figur 8 jede Schicht 109, 209 auf einer eigenen Hülse 15 angeordnet. Hierdurch wird eine HF-Kopplung innerhalb einer einzigen Hülse im Bereich des Abstandes L vermindert. Selbstverständlich können auch hier wieder die Öffnungen 11 und 14 vorgesehen sein.

Die Erfindung kann vorteilhaft auch bei Mehrlumenkathetern angewandt oder zugleich bei solchen eingesetzt werden, mit denen eine Ultraschall- oder Laser- oder eine mechanische Behandlung möglich ist. Insbesondere ist die Erfindung mit Vorteil anwendbar bei einem Doppeloder Mehrfachkatheter mit insbesondere kontinuierlich in der Länge veränderbarem Behandlungsbereich gemäß der deutschen Patentanmeldung P 34 06 294.7. Insbesondere kann der erfindungsgemäße Katheter in einen bekannten Katheter eingeschoben und an dessen vorderem Ende für eine gezielte Behandlung herausgeschoben werden.

Der Erwärmungsgrad und die Dauer der Einwirkung werden durch geeignete Wahl der eingekoppelten Leistung und der Dauer derselben eingestellt. Auch ist es möglich, die Isttemperatur im Behandlungsbereich über geeignete Senszoren zu messen und über eine Regelstrecke eine vorgegebene Leistungskurve zu durchfahren. Die Energierzufuhr erfolgt also entweder gemäß einem Leistungs-Zeit-Diagramm oder entsprechend einem t°-Zeit-Diagramm werden die Leistung und/oder die Zeit nachgesteuert oder nachgeregelt.

Mit dem erfindungsgemäßen Katheter mit den Ballon-Ausführungsformen gemäß Figuren 1, 3, 5 und 7 kann insbesondere die Ballondilatation in dem Sinne verbessert werden, daß die bisher häufig aufgetretenen Restenosierungen im Bereich der dilatierten Areale weitestgehend vermieden werden, da mittels der verwendeten elektrischen Energie, insbesondere Hochfrequenzenergie, die bisher lediglich mechanisch erzeugten Gefäßaufweitungen auf Grund von Erwärmungs-, Verdampfungs- oder Koagulierungsvorgängen stabilisiert werden.

In Figur 9 ist schließlich noch eine weitere Ballon-Ausführungsform eines erfindungsgemäßen Katheters dargestellt, die sich von dem Katheter 1 gemäß Figur 1 nur dadurch unterscheidet, daß zwischen dem aufblasbaren Ballon 8 und der elektrisch leitenden Schicht 9 eine Zwischenschicht 20 aus dielektrischem, schlecht wärmeleitendem Material angeordnet ist. Die Zwischenschicht 20 besteht vorzugsweise aus Magnesiumfluorid. Die dielektrische Zwischenschicht ermöglicht die Anwendung höherer HF-Energiekonzentrationen, weil mit dieser Zwischenschicht 20 der Ballon 8 vor übermäßiger Erwärmung auch in diesem Falle geschützt ist.

Im übrigen können auch bei der Ausführungsform gemäß Figur 9 im Ballon 8, in der elektrisch leitenden Schicht 9 und in der Zwischenschicht 20 Öffnungen 14 vorgesehen sein, wie sie anhand der früher erläuterten Ausführungsbeispiele beschrieben wurden. Auch bei den anderen bereits beschriebenen Katherern mit abdichtendem Ballon können entsprechende Zwischenschichten vorgesehen sein.

Bei den Katheterausführungen mit abdichtendem Ballon 8 wird die elektrisch leitende Schicht 9, 109, 209 im vollaufgeblasenen Zustand des Ballons 8 vorzugsweise durch Aufdampfen aufgebracht.

Wie beschrieben, können sowohl monopolare also auch bipolare Elektrodenanordnungen verwendet werden. Die Zuführung von HF-Energie in dünnen Kathetern ist für das monopolare Verfahren einfacher, wobei allerdings die Eindringtiefe der elektrischen Energie geringer ist als bei den früher beschriebenen bipolaren Elektrodenanordnungen. Allerdings ist die Temperaturverteilung bei den bipolaren Elektrodenanordnungen übersichtlicher und leichter meßbar.

Die Kombination von Dilatation und Koagulation, evtl. unter Verwendung steriler Physiologischer Kochsalzlösung als druckübertragendes Medium und die Einbringung von Spülöffnungen in den Ballon bringt folgende zusätzliche Vorteile:

Es wird eine vollstände Verdrängung des Blutes zwischen Ballon und Gefäßwand erreicht. Ferner verhindert die physiologische Kochsalzlösung die Verklebung zwischen dem Ballon und der Gefäßwand. Der Kontakt der Elektroden zur Gefäßwand über den Ballondruck bringt reproduzierbare Verhältnisse auch bei unvermeidbaren physiologischen Bewegungen und schafft ohne Spülung eine praktisch trockene Gewebeoberfläche.

Die Frequenzen zur intravenösen Koagulation biologischer Materialien, wie Stenosematerialien, liegen günstigerweise zwischen 300 kHz und 3MHz. In diesem Frequenzbereich reich tritt im Strömungsfeld keine Reizwirkung auf den Patienten, sondern lediglich eine Gewebeerhitzung auf. Grundsätzlich kann - wie bereits erwähnt - die Erhitzung der metallischen Koagulationselektrode auch durch ein externes HF-Feld erzeugt werden. Hierbei entsteht die Wärme durch Wirbelströme, die in leitenden Werkstoffen durch Induktion einer Spannung mit einem magnetischen Wechselfeld erzeugt werden. Das Magnetfeld läuft parallel zur Spulenachse. Hierbei können mit relativ geringer HF-Energie in kurzen Aufheizzeiten relativ hohe Temperaturen in dem Dilatationsleiter im Gefäß erreicht werden.

**Patentansprüche**

1. Katheter mit wenigstens einem am vorderen Ende (3) geschlossenen Lumen (12) zur lokalen Behandlung innerer Körperstrukturen, insbesondere Stenosen, mit wenigstens einer in einem am vorderen Ende (3) zur Behandlung vorgesehenen Abschnitt (B) angebrachten Öffnung (11) in der Katheterwandung (7), die vom Lumen (12) bis zur Außenwand des Katheters (1) reicht, dadurch gekennzeichnet, daß im Behandlungsabschnitt (B) des Katheterendes (3) ein dehnbares und aufweitbares Teil (8, 15) des Katheters (1) vorgesehen ist, auf dessen Außenfläche zumindest ein die Ausdehnung dieses Katheterteiles (8, 15) mitmachender elektrisch leitender Bereich aufgebracht oder dieser Katheterteil (8, 15) selbst in zumindest einem Bereich elektrisch leitend ausgebildet ist.

2. Katheter nach Anspruch 1, dadurch gekennzeichnet, daß der elektrisch leitende Bereich als elektrisch leitende Schicht (9, 109, 209) ausgebildet ist.

3. Katheter nach Anspruch 1, dadurch gekennzeichnet, daß der elektrisch leitende Bereich auf einer elastischen Hülse (15) vorgesehen ist, die den Katheter (1) zumindest teilweise koaxial umgibt und wenigstens eine Öffnung (14) aufweist.

4. Katheter nach Anspruch 3, dadurch gekennzeichnet, daß die elastische Hülse (15) aus schlecht wärmeleitfähigem Material besteht.

5. Katheter nach Anspruch 3 oder 4, dadurch gekennzeichnet, daß die elastische Hülse (15) aus elektrisch leitfähigem Material besteht.

6. Katheter nach Anspruch 1 oder 2, dadurch gekennzeichnet, daß der elektrisch leitende Bereich (9, 109,209) auf einem den Katheter (1) im Behandlungsabschnitt (B) umgebenden aufblasbaren Ballon (8) aufgebracht ist.

7. Katheter nach Anspruch 6, dadurch gekennzeichnet, daß der durch ein Druckmittel aufweitbare Ballon (8) im Behandlungsabschnitt (B) mit wenigstens einer Öffnung (14) in der Ballonwand (13) für den Austritt des Druckmittels versehen ist.

8. Katheter nach Anspruch 6 oder 7, dadurch gekennzeichnet, daß die Katheterwand (7) im Bereich des Ballons (8) eine oder mehrere Öffnungen (11) aufweist, deren Querschnitt bzw. Gesamtquerschnitt größer ist als der Querschnitt bzw. Gesamtquerschnitt der Öffnung bzw.Öffnungen (14) in der Ballonwand (13).

9. Katheter nach einem der Ansprüche 1 bis 8, dadurch gekennzeichnet, daß auf dem Katheter (1), im Lumen des Katheters (1) oder in der Katheterwand (7) eine elektrische Zuleitung (16, 116, 216) vom hinteren Katheterende zum elektrisch leitenden Bereich (9, 109, 209) vorgesehen ist.

10. Katheter nach Anspruch 9, dadurch gekennzeichnet, daß bei Anordnung der Zuleitung (16, 116, 216) außen auf dem Katheter (1) dieser zumindest im Anschlußbereich zur leitenden Schicht (9, 109, 209) nach außen mit einer Isolierschicht (19) versehen ist.

11. Katheter nach Anspruch 9 oder 10, dadurch gekennzeichnet, daß die Zuleitung (16, 116, 216) eine Koaxialleitung ist.

12. Katheter nach einem der Ansprüche 9 bis 11, dadurch gekennzeichnet, daß zumindest eine innere Zuleitung (16, 116, 216) über leitende, insbesondere metallisierte Bohrungswandungen der Öffnungen (14) mit der elektrisch leitenden Schicht (9, 109, 209) elektrisch leitend verbunden ist.

13. Katheter nach einem der Ansprüche 9 bis 12, dadurch gekennzeichnet, daß die oder eine innere Zuleitung (16, 116, 216) bis zum vorderen Ende (3) des Katheters (1) geführt und dort nach außen isoliert zurückgeführt und mit der leitenden Schicht (9, 109, 209) elektrisch leitend verbunden ist.

14. Katheter nach einem der Ansprüche 9 bis 13, dadurch gekennzeichnet, daß axial hintereinander zwei im Abstand (L) voneinander angeordnete leitende Schichten (109, 209) eine bipolare Anordnung bilden und jede mit einer eigenen Zuleitung (116, 216) elektrisch leitend verbunden ist.

15. Katheter nach einem der Ansprüche 1 bis 14, dadurch gekennzeichnet, daß das vordere Ende (3) des Katheters (1) mit einem elektrisch leitenden Einsatz (4) verschlossen ist.

16. Katheter nach Anspruch 15, dadurch gekennzeichnet, daß der Einsatz (4) mit sowohl einer Zuleitung (16, 216) als auch mit der bzw. einer leitenden Schicht (9, 209) elektrisch leitend verbunden ist.

17. Katheter nach einem der Ansprüche 14 bis 16, durch gekennzeichnet, daß die axiale Länge jeder leitende leitenden Schicht (109, 209) etwa dem Abstand (L) zwischen den benachbarten Schichten (109, 209) entspricht.

18. Katheter nach einem der Ansprüche 6 bis 17, dadurch gekennzeichnet, daß zwischen dem aufblasbaren Ballon (8) und der elektrisch leitenden Schicht (9, 109, 209) eine Zwischenschicht (20) aus dielektrischem Material angeordnet ist.

19. Katheter nach Anspruch 18, dadurch gekennzeichnet, daß die Zwischenschicht (20) aus Magnesiumfluorid besteht.

**Claims**

1. A catheter comprising at least one lumen, closed at the front end (3), for the local treatment of internal body structures, in particular stenoses, comprising at least one opening (11) in the catheter wall (7) which is arranged in a treatment section (B) at the front end (3) and which extends from the lumen (12) to the outer wall of the catheter (1), characterised in that an extendable and expandable part (8, 15) of the catheter (1) is arranged in the treatment section (B) of the catheter end (3), at least one electrically conductive zone which forms part of the extension of this part (8, 15) of the catheter being arranged on the outer surface of this part of the catheter, or this part (8, 15) of the catheter itself being electrically conductive in at least one zone.

2. A catheter as claimed in claim 1, characterised in that the electrically conductive zone is in the form of an electrically conductive coating (9, 109, 209).

3. A catheter as claimed in claim 1, characterised in that the electrically conductive zone is arranged on an elastic sleeve (15) which at least partially coaxially surrounds the catheter (1) and has at least one opening (14).

4. A catheter as claimed in claim 3, characterised in that the elastic sleeve (15) consists of a poor heat conducting material.

5. A catheter as claimed in claim 3 or claim 4, characterised in that the elastic sleeve (15) consists of electrically conductive material.

6. A catheter as claimed in claim 1 or claim 2, characterised in that the electrically conductive zone (9, 109, 209) is arranged on an inflatable balloon (8) which surrounds the catheter (1) in the treatment section (B).

7. A catheter as claimed in claim 6, characterised in that the balloon (8) which can be expanded by a pressure means and which is arranged in the treatment section (B) is provided with at least one opening (14) in the balloon wall (13) for the outlet of the pressure means.

8. A catheter as claimed in claim 6 or claim 7, characterised in that in the region of the balloon (8) the catheter wall (7) has one or more opening(s) (11) whose cross-section or overall cross-section is greater than the cross-section or overall cross-section of the opening or openings (14) in the balloon wall (13).

9. A catheter as claimed in one of the claims 1 to 8, characterised in that an electrical supply line (16, 116, 216) from the rear end of the catheter to the electrically conductive zone (9, 109, 209, is arranged on the catheter (1), in the lumen of the catheter (1) or in the catheter wall (7).

10. A catheter as claimed in claim 9, characterised in that when the supply line (16, 116, 216) is arranged on the exterior of the catheter (1), the catheter is provided with an external insulating coating (19) at least in the area in which it is concerted to the conductive coating (9, 109, 209).

11. A catheter as claimed in claim 9 or claim 10, characterised in that the supply line (16, 116, 216) is a coaxial line.

12. A catheter as claimed in one of the claims 9 to 11, characterised in that at least one inner supply line (16, 116, 216) is electrically conductively connected to the electrically conductive coating (9, 109, 209) via conductive, in particular metallised, bore walls of the openings (14).

13. A catheter as claimed in one of the claims 9 to 12, characterised in that the or an inner supply line (16, 116, 216) extends to the front end (3) of the catheter (1) from where, with external insulation, it is fed back and electrically conductively connected to the conductive coating (9, 109, 209).

14. A catheter as claimed in one of the claims 9 to 13, characterised in that axially in series two conductive coatings (109, 209) arranged at a distance (L) from one another form a bipolar arrangement and each is electrically conductively connected to a separate supply line (116, 216).

15. A catheter as claimed in one of the claims 1 to 14, characterised in that the front end (3) of the catheter (1) is closed by an electrically conductive insert (4).

16. A catheter as claimed in claim 15, characterised in that the insert (4) is electrically conductively connected both to a supply line (16, 216) and to the or a conductive coating (9, 209).

17. A catheter as claimed in one of the claims 14 to 16, characterised in that the axial length of each conductive coating (109, 209) corresponds approximately to the distance (L) between the adjacent coatings (109, 209).

18. A catheter as claimed in one of the claims 6 to 17, characterised in that an intermediate layer (20) composed of dielectric material is arranged between the inflatable balloon (8) and the electrically conductive coating (9, 109, 209).

19. A catheter as claimed in claim 18, characterised in that the intermediate layer (20) consists of magnesium fluoride.

**Revendications**

1.- Cathéter avec au moins un lumen fermé à l'extrémité avant (1), pour traitement local de structures internes du corps, en particulier des sténoses, avec dans la paroi du cathéter (7) sur un tronçon (B) prévu pour traitement, à son extrémité avant (3), au moins une ouverture allant du lumen (12) jusqu'à la paroi extérieure du cathéter, caractérisé en ce que dans le segment de traitement (B) de l'extrémité (3) du cathéter est prévue une partie élastique et extensible (8, 15) du cathéter, sur la surface de laquelle est introduite au moins une zone conductrice de l'électricité faisant partie de l'étendue de cette partie de cathéter (8, 15) ou bien cette partie de cathéter (8, 15) elle-même est réalisée conductrice de l'électricité dans au moins une zone.

2.- Cathéter selon la revendication 1, caractérisé en ce que la zone conductrice de l'électricité est réalisée comme une couche conductrice de l'électricité (9, 109, 209).

3.- Cathéter selon la revendication 1, caractérisé en ce que la zone conductrice de l'électricité est prévue sous forme d'une gaine élastique (15) qui entoure coaxialement le cathéter (1) au moins en partie et comporte au moins une ouverture (14).

4.- Cathéter selon la revendication 3, caractérisé en ce que la gaine (15) élastique est composée de matériau mauvais conducteur de la chaleur.

5.- Cathéter selon les revendications 3 ou 4, caractérisé en ce que la gaine élastique (15) est composée de matériau conducteur de l'électricité.

6.- Cathéter selon les revendications 1 ou 2, caractérisé en ce que la zone conductrice de l'électricité (9, 109, 209) est introduite sur un ballon (8) gonflable entourant le cathéter (1) dans le tronçon de traitement (B).

7.- Cathéter selon la revendication 6, caractérisé en ce que le ballon (8) extensible par un agent de pression, est muni, dans le tronçon de traitement (B), d'au moins une ouverture (14) dans la paroi du ballon (13) pour la sortie de l'agent de pression.

8.- Cathéter selon les revendications 6 ou 7, caractérisé en ce que la paroi du cathéter (7), dans la zone du ballon (8), présente une ou plusieurs ouvertures (11), dont la section transversale ou section transversale totale est plus grande que la section transversale ou section transversale totale de l'ouverture ou des ouvertures (14) dans la paroi du ballon (13).

9.- Cathéter selon l'une quelconque des revendications 1 à 8, caractérisé en ce que sur le cathéter (1), dans le lumen du cathéter (1) ou dans la paroi du cathéter (7), un conducteur d'amenée électrique (16, 116, 216) est prévu depuis l'extrémité arrière du cathéter jusqu'à la zone conductrice de l'électricité (9, 109, 209).

10.- Cathéter selon la revendication 9, caractérisé en ce que par mise en place du conducteur (16, 106, 206) à l'extérieur sur le cathéter (1), celui-ci, au moins dans la zone de raccordement avec la couche conductrice (9, 109, 209), est muni vers l'extérieur d'une couche isolante (19).

11.- Cathéter selon les revendications 9 ou 10, caractérisé en ce que le conducteur (16, 116, 206) est un conducteur coaxial.

12.- Cathéter selon l'une quelconque des revendications 9 à 11, caractérisé en ce qu'au moins un conducteur intérieur (16, 116, 216) est relié au conducteur électrique avec la couche conductrice (9, 109, 209) par les parois conductrices, notamment métallisées du trou des ouvertures (14).

13.- Cathéter selon l'une quelconque des revendications 9 à 12, caractérisé en ce que le ou un conducteur intérieur (16, 116, 216) est amené jusqu'à l'extrémité avant (3) du cathéter et là est ramené, avec isolement, vers l'extérieur et est connecté avec conduction électrique sur la couche conductrice (9, 109, 209).

14.- Cathéter selon l'une quelconque des revendications 9 à 13, caractérisé en ce qu'axialement l'une derrière l'autre, deux couches (109, 209) conductrices, placées à distance (L) l'une de l'autre, forment une disposition bipolaire, et chacune est connectée en conduction électrique avec un conducteur propre (116, 216).

15.- Cathéter selon l'une quelconque des revendications 1 à 14, caractérisé en ce que l'extrémité avant (3) du cathéter (1) est enfermée dans un revêtement (4) conducteur de l'électricité.

16.- Cathéter selon la revendication 15, caractérisé en ce que le revêtement (4) est connecté en conduction électrique aussi bien avec un conducteur (16, 216) qu'avec la ou une couche conductrice (9, 209).

17.- Cathéter selon l'une quelconque des revendications 14 à 16, caractérisé en ce que la longueur axiale de chaque couche conductrice (109, 209) correspond à peu près à la distance (L) entre les couches avoisinantes (109, 209).

18.- Cathéter selon l'une quelconque des revendications 6 à 17, caractérisé en ce que, entre le ballon (8) gonflable et la couche conductrice de l'électricité (9, 109, 209), est mise en place une couche intermédiaire (20) en matériau diélectrique.

19.- Cathéter selon la revendication 18, caractérisé en ce que la couche intermédiaire (20) est composée de fluorure de magnésium.

EP 0 205 851 B1

Fig.1

Fig.2

Fig.3

Fig.4

Fig.5

Fig.6

Fig.7

Fig.8

EP 0 205 851 B1

Fig.9